Europäisches Patentamt

European Patent Office

Office européen des brevets

(11) Veröffentlichungsnummer : **0 033 851**
**B1**

(12) **EUROPÄISCHE PATENTSCHRIFT**

(45) Veröffentlichungstag der Patentschrift :
09.03.83

(21) Anmeldenummer : 81100258.3

(22) Anmeldetag : 15.01.81

(51) Int. Cl.³ : **C 07 C 55/14, C 07 C 51/44**

(54) Verfahren zur Abtrennung von Adipinsäure aus Gemischen mit Glutarsäuren und/oder Bernsteinsäuren.

(30) Priorität : 23.01.80 DE 3002256

(43) Veröffentlichungstag der Anmeldung :
19.08.81 Patentblatt 81/33

(45) Bekanntmachung des Hinweises auf die Patenterteilung : 09.03.83 Patentblatt 83/10

(84) Benannte Vertragsstaaten :
BE CH DE FR GB IT LI NL

(56) Entgegenhaltungen :
DE A 2 151 535
DE B 1 272 913

(73) Patentinhaber : BASF Aktiengesellschaft
Carl-Bosch-Strasse 38
D-6700 Ludwigshafen (DE)

(72) Erfinder : Rebafka, Walter, Dr.
Schuetzenstrasse 4
D-6901 Eppelheim (DE)
Erfinder : Hellen, Gerd, Dr.
Schifferstadter Strasse 1B
D-6720 Speyer (DE)
Erfinder : Klink, Walter, Dr.
Schlesierstrasse 11
D-6701 Birkenheide (DE)

Verfahren zur Abtrennung von Adipinsäure aus Gemischen mit Glutarsäuren und/oder Bernsteinsäuren.

Gegenstand der Erfindung ist ein Verfahren zur Abtrennung von Adipinsäure aus solche enthaltenen Gemischen mit Glutarsäuren und/oder Bernsteinsäuren, wobei man die Glutarsäuren und Bernsteinsäuren mit Stickstoffbasen bei erhöhter Temperatur zu Glutarsäureimiden und Succinimiden umsetzt und diese durch Destillation abtrennt.

Bei der Herstellung von Adipinsäure aus einem Gemisch von Cyclohexanol und Cyclohexanon durch Oxidation mit Salpetersäure erhält man als Nebenprodukt ein Gemisch aus Adipinsäure, Glutarsäure und Bernsteinsäure. Solche Gemische werden beispielsweise beschrieben in der DE-OS 20 51 320. Adipinsäure, Glutarsäure und Bernsteinsäure enthaltende Gemische erhält man auch durch Oxidation von Caprolactamoligomeren mit Salpetersäure, wie aus der DE-OS 25 02 837 bekannt ist. Gemische aus Adipinsäure, α-Methylglutarsäure und — Äthylbernsteinsäure erhält man durch Umsetzen von Butadien mit Kohlenmonoxid und Wasser in Gegenwart von Kobaltcarbonylkatalysatoren und Pyridin, entsprechend der DE-OS 1 618 156. Solche Säuregemische können zwar als Säuerungsmittel in der Textil — und Lederindustrie verwendet werden, man ist jedoch bestrebt, die wertvolle Adipinsäure aus solchen Gemischen zu gewinnen. Dies erfordert jedoch eine aufwendige mehrstufige Destillation. Aus der BE-PS 773 931 ist auch schon bekannt, daß man die in einem solchen Gemisch enthaltene Bernsteinsäure und Glutarsäure mit Ammoniak zu den entsprechenden Säureimiden umsetzt und von der verbleibenden Adipinsäure abdestilliert. Dieses Verfahren ist jedoch noch verbesserungsbedürftig, da noch erhebliche Mengen an Glutarsäure und Bernsteinsäure in der Adipinsäure verbleiben.

Es war deshalb die technische Aufgabe gestellt, die Abtrennung von Adipinsäure aus solche enthaltenden Gemischen mit Glutarsäuren und Bernsteinsäuren wirkungsvoller zu gestalten und zugleich Glutarsäure und Bernsteinsäure in Form ihrer Imide als Höherwertige Produkte zu erhalten.

Diese Aufgabe wird gelöst in einem Verfahren zur Abtrennung von Adipinsäure aus solche enthaltenden Gemischen mit Glutarsäuren und/oder Bernsteinsäuren, wobei man die Glutarsäuren und Bernsteinsäuren mit Stickstoffbasen bei erhöhter Temperatur zu Glutarsäureimiden und Succinimiden umsetzt und diese durch Destillation abtrennt, wobei man als Stickstoffbase primäre $C_1$- bis $C_6$-Alkylamine verwendet.

Das neue Verfahren hat den Vorteil, daß die Abtrennung der Adipinsäure auf einfache Weise vollständiger als bisher gelingt. Ferner hat das neue Verfahren den Vorteil, daß man wertvolle N-Alkylsuccinimide und N-Alkylglutarimide erhält.

Geeignete Ausgangsgemische enthalten neben Adipinsäure Glutarsäure oder α-Methylglutarsäure und Bernsteinsäure oder Äthylbernsteinsäure.

Bevorzugte Gemische enthalten neben Adipinsäure Glutarsäure und Bernsteinsäure. Typische Gemische enthalten 10 bis 70 Gew.% Adipinsäure, 20 bis 70 Gew.% Glutarsäure und 10 bis 50 Gew.% Bernsteinsäure. Solche Gemische können von der Herstellung her noch Metallsalze, wie Vanadium- oder Kupfersalze enthalten. Geeignete Gemische fallen als Nebenprodukte bei der Herstellung von Adipinsäure an, wie aus der DE-OS 20 51 320 zu entnehmen ist. Geeignete Gemische erhält man auch durch Oxidation von Caprolactamoligomeren mit Salpetersäure entsprechend DE-OS 25 02 837. Ferner erhält man Gemische aus Adipinsäure, α-Methylglutarsäure und Äthylbernsteinsäure nach dem in der DE-OS 16 18 156 beschriebenen Verfahren.

Geeignete Amine sind primäre $C_1$- bis $C_6$-Alkylamine wie Methylamin, Äthylamin, Propylamin, Isopropylamin, n-Butylamin oder n-Hexylamin. Die Amine können als solche oder z. B. als wäßrige Lösung angewandt werden. Besondere technische Bedeutung hat Methyl- und Äthylamin erlangt. Zweckmäßig wendet man je Mol Glutar- und Bernsteinsäure, die im Säuregemisch enthalten sind, 1 Mol Alkylamine an. Ein geringes Überschreiten der stöchiometrischen Menge, z. B. bis zu 5 Mol% ist zweckmäßig um auch bei nichtvollständigen Umsatz der primären Amine eine vollständige Umsetzung der Glutar- bzw. Bernsteinsäuren zu gewährleisten.

Vorteilhaft führt man die Umsetzung bei einer Temperatur von 100 bis 190 °C durch. Es hat sich besonders bewährt, wenn man das Säuregemisch und die Amine bei einer Temperatur von 100 bis 150 °C mischt und dann das Gemisch auf 160 bis 190 °C erhitzt. Die Umsetzung wird zweckmäßig unter Normaldruck oder schwach erhöhtem Druck, z. B. bis zu 5 bar, durchgeführt. Es ist möglich, zum vorgelegten aufgeschmolzenen Säuregemisch Alkylamine zuzugeben oder umgekehrt, die Alkylamine gegebenenfalls in einem Lösungsmittel vorzulegen und das Säuregemisch geschmolzen oder in Lösung zuzugeben. Während der Reaktion destilliert man zweckmäßig die bei der Reaktionstemperatur flüchtigen Anteile fortlaufend ab.

Die Abtrennung der gebildeten N-Alkylimide der Glutar- und Bernsteinsäure von der nicht umgesetzten Adipinsäure erfolgt durch Destillation. In der Regel genügt eine einstufige Destillation. Die Destillation kann unter Normaldruck durchgeführt werden. Aufgrund der hohen Siedepunkte ist es doch zweckmäßig, unter vermindertem Druck zu arbeiten.

Das Verfahren nach der Erfindung ist insofern bemerkenswert als die primären Amine nur mit der im Gemisch enthaltenen Bernsteinsäure und Glutarsäure reagieren, während die Adipinsäure nicht angegriffen wird. Bei Kenntnis der BE-PS 773 931 konnte dies nicht erwartet werden, da primäre Amine nukleophiler und damit reaktiver sind als Ammoniak. Es war im Vergleich zu Ammoniak bei der Verwendung von primären Aminen eine deutlich unselektivere Umsetzung zu erwarten gewesen.

Die nach dem erfindungsgemäßen Verfahren gewonnene Adipinsäure kann für die meisten Anwendungen ohne weitere Aufarbeitung verwendet werden. Es ist jedoch auch möglich, die so zurückgewonnene Adipinsäure der bei der Oxidation von Cyclohexanon oder Cyclohexanol anfallenden Adipinsäure zuzugeben und diese gemeinsam aufzuarbeiten.

Die nach dem Verfahren der Erfindung erhaltenen N-Alkylimide der Glutar- und Bernsteinsäure eignen sich als Lösungsmittel, als Oxidationsstabilisatoren oder gegebenenfalls nach. Trennung durch Destillation als Zwischenprodukt für die Herstellung von Farbstoffen und Pflanzenschutzmittel.

Das Verfahren nach der Erfindung sei an folgenden Beispielen veranschaulicht.

Beispiel 1

In einem 600-l-Reaktionskessel mit Rührer und aufgesetzter Destillationsvorrichtung werden 374 kg eines Säuregemisches vorgelegt, das aus 19,3 Gew.% Bernsteinsäure, 48,2 Gew.% Glutarsäure und 29,8 Gew.% Adipinsäure besteht. Dieses Gemisch enthält zusätzlich 0,39 Gew.% Kupfer und 0,12 Gew.% Vanadium in Form von Salzen. Der Kessel wird auf 120 °C aufgeheizt und bei dieser Temperatur werden innerhalb von 5 Stunden 156 kg einer 40 %igen wäßrigen Monomethylamin-Lösung in die Säureschmelze gepumpt. Nach beendetem Zulauf wird die Kesseltemperatur auf 160 °C unter Abdestillation der flüchtigen Bestandteile erhöht. Das anfallende Destillat besteht im wesentlichen aus Wasser und geringen Mengen an nicht umgesetztem Monomethylamin. Anschließend wird bei einem von 15 mbar und einer Kesseltemperatur von 180 °C das gebildete Gemisch aus N-Methylglutarimid und N-Methylsuccinimid abdestilliert. Man erhält 251 kg Destillat, bestehend aus 69 Gew.% N-Methylglutarsäureimid, 28 Gew.% N-Methylsuccinimid und 3 Gew.% Wasser. In dieser Fraktion konnte keine Adipinsäure, weder als freie Säure noch als Adipinsäureimid, nachgewiesen werden.

Im Reaktionskessel verbleibt ein Rückstand von 114 kg. Die Säurezahl des Rückstandes (Verbrauch an KOH in mg/g Substanz) beträgt 746, was einem Gehalt von 97,3 % entspricht (berechnet als Adipinsäure). Eine Probe dieses Rückstandes wird zur Bestimmung der Glutar- bzw. Bernsteinsäureanteile mit methanolischer Salzsäure verestert. Der gebildete Methylester besteht nach gaschromatographischer Analyse zu 99,7 % aus Adipinsäuredimethylester zu zu 0,3 % aus Glutarsäuredimethylester, während kein Bernsteinsäuredimethylester nachweisbar ist.

Beispiel 2

Man verfährt wie im Beispiel 1 beschrieben und setzt 74 kg Äthylamin mit 380 kg eines Säuregemisches um, das zu 43 % aus Adipinsäure, zu 40 % aus Glutarsäure, zu 15 % aus Bernsteinsäure besteht und keine Metallsalze enthält.

Unter Reaktionsbedingungen, die denen des Beispiels 1 entsprechen, wird nach Abdestillation des Reaktionswassers ein Destillat von 230 kg erhalten, welches zu 27 % aus N-Äthylsuccinimid, zu 70 % aus N-Äthylglutarimid und zu 3 % aus Wasser besteht.

Der Rückstand hat eine Säurezahl von 751. Der Gehalt an Adipinsäure (bestimmt mittels Analyse der Methylester wie im Beispiel 1) beträgt 99 %, die entsprechend berechneten Anteile an Glutar- bzw. Bernsteinsäure betragen 0,7 bzw. 0,3 %

**Ansprüche**

1. Verfahren zur Abtrennung von Adipinsäure aus solche enthaltenen Gemischen mit Glutarsäuren und/oder Bernsteinsäuren, wobei man die Glutarsäuren und Bernsteinsäuren mit Stickstoffbasen bei erhöhter Temperatur zu Glutarsäureimiden und Succinimiden umsetzt und diese durch Destillation abtrennt, dadurch gekennzeichnet, daß man als Stickstoffbasen primäre $C_1$- bis $C_6$-Alkylamine verwendet.

2. Verfahren nach Anspruch 1, dadurch gekennzeichnet, daß man je Mol der im Gemisch enthaltenen Glutarsäuren und Bernsteinsäuren 1 Mol primäre Alkylamine anwendet.

3. Verfahren nach den Ansprüchen 1 und 2, dadurch gekennzeichnet, daß man die Umsetzung bei 100 bis 190 °C durchführt.

4. Verfahren nach den Ansprüchen 1 bis 3, dadurch gekennzeichnet, daß man das Säuregemisch und die Amine bei einer Temperatur von 100 bis 150 °C mischt und dann auf 160 bis 190 °C unter Abdestillieren der flüchtigen Anteile erhitzt.

5. Verfahren nach den Ansprüchen 1 bis 4, dadurch gekennzeichnet, daß man ein Gemisch aus 10 bis 70 Gew.% Adipinsäure, 20 bis 70 Gew.% Glutarsäure und 10 bis 50 Gew.% Bernsteinsäure verwendet.

6. Verfahren nach den Ansprüchen 1 bis 5, dadurch gekennzeichnet, daß man Methylamin oder Äthylamin verwendet.

**Claims**

1. A process for isolating adipic acid from a mixture of adipic acid with glutaric acids and/or succinic acids, the glutaric acids and succinic acids being reacted with a nitrogen base at an elevated temperature to give glutarimides and succinimides which are removed by distillation, wherein a primary $C_1$-$C_6$-alkylamine is used as the nitrogen base.

2. A process as claimed in claim 1, wherein 1 mole of primary alkylamine is used per mole of glutaric acids and succinic acids contained in the mixture.

3. A process as claimed in claims 1 and 2, wherein the reaction is carried out at from 100 to 190 °C.

4. A process as claimed in claims 1 to 3, wherein the acid mixture and the amine are mixed at 100-150 °C and then heated to 160-190 °C, whilst distilling off the volatile constituents.

5. A process as claimed in claims 1 to 4, wherein a mixture of 10-70 % by weight of adipic acid, 20-70 % by weight of glutaric acid and 10-50 % by weight of succinic acid is used.

6. A process as claimed in claims 1 to 5, wherein methylamine or ethylamine is used.

**Revendications**

1. Procédé pour séparer l'acide adipique de mélanges contenant celui-ci avec des acides glutariques et/ou succiniques, les acides glutariques et succiniques étant transformés, avec des bases azotées, à une température élevée, en imides d'acides glutariques et succinimides, qui sont séparés par distillation, caractérisé par le fait qu'on utilise, comme bases azotées, des alkylamines primaires en $C_1$ à $C_6$.

2. Procédé selon la revendication 1, caractérisé par le fait qu'on utilise 1 mole d'alkylamine primaire par mole d'acides glutariques et succiniques contenus dans le mélange.

3. Procédé selon les revendications 1 et 2, caractérisé par le fait qu'on effectue la réaction à une température de 100 à 190 °C.

4. Procédé selon les revendications 1 à 3, caractérisé par le fait qu'on mélange le mélange d'acides et l'amine à une température de 100 à 150 °C puis on élève la température jusqu'à 160 à 190 °C, en éliminant par distillation les parties volatiles.

5. Procédé selon les revendications 1 à 4, caractérisé par le fait qu'on part d'un mélange de 10 à 70 % en poids d'acide adipique, 20 à 70 % en poids d'acide glutarique et 10 à 50 % en poids d'acide succinique.

6. Procédé selon les revendications 1 à 5, caractérisé par le fait qu'on utilise de la méthylamine ou de l'éthylamine.